Europäisches Patentamt

European Patent Office

Office européen des brevets

(19)

(11) EP 0 752 101 B1

(12) **EUROPÄISCHE PATENTSCHRIFT**

(45) Veröffentlichungstag und Bekanntmachung des Hinweises auf die Patenterteilung:
**18.11.1998 Patentblatt 1998/47**

(51) Int Cl.[6]: **G01N 33/18**, G01N 33/15, G01N 33/02

(21) Anmeldenummer: **95910385.4**

(22) Anmeldetag: **13.03.1995**

(86) Internationale Anmeldenummer:
**PCT/CH95/00055**

(87) Internationale Veröffentlichungsnummer:
**WO 95/25955 (28.09.1995 Gazette 1995/41)**

(54) **VERFAHREN UND VORRICHTUNG ZUR BESTIMMUNG DER TOXIZITÄT SOWIE DEREN ANWENDUNG**

METHOD AND DEVICE FOR DETERMINING TOXICITY AND APPLICATION THEREOF

PROCEDE ET DISPOSITIF POUR DETERMINER LA TOXICITE, ET APPLICATION

(84) Benannte Vertragsstaaten:
**AT BE CH DE DK ES FR GB GR IE IT LI LU MC NL PT SE**

(30) Priorität: **19.03.1994 CH 811/94**

(43) Veröffentlichungstag der Anmeldung:
**08.01.1997 Patentblatt 1997/02**

(73) Patentinhaber: **Schweizerische Eidgenossenschaft**
**vertreten durch das AC-Laboratorium Spiez der Gruppe für Rüstungsdienste**
**CH-3700 Spiez (CH)**

(72) Erfinder:
 • **PORTMANN, Rudolf**
  **CH-3000 Bern 23 (CH)**
 • **LEUMANN, Mathias**
  **CH-8484 Weisslingen (CH)**
 • **THOMMEN, Stefan**
  **CH-8302 Kloten (DE)**

(74) Vertreter: **Frauenknecht, Alois J. et al**
**c/o PPS Polyvalent Patent Service AG,**
**Waldrütistrasse 21**
**8954 Geroldswil (CH)**

(56) Entgegenhaltungen:
 **WO-A-90/12886         DE-A- 2 906 194**
 **DE-A- 3 345 196        DE-A- 3 922 358**

 • **DATABASE WPI Section 768, Week 3192 Derwent Publications Ltd., London, GB; AN 92-258109 C31! & SU,A,1 677 630 (MED TECH RES INST) , 15.September 1991**
 • **REVIEW OF SCIENTIFIC INSTRUMENTS., Bd. 62, NEW YORK US, Seite 540 SHOJI A. BABA, ET AL. 'Three-dimensional recording and measurement of swimming paths of micro-organisms with two synchronized monochrome cameras'**
 • **PATENT ABSTRACTS OF JAPAN vol. 13 no. 245 (P-881) ,8.Juni 1989 & JP,A,01 047950 (RES DEV CORP OF JAPAN) 22.Februar 1989,**

**Beschreibung**

Die Erfindung betrifft eine Vorrichtung gemäss dem Oberbegriff des Patentanspruchs 1.

Ebenso bezieht sich die Erfindung auf Verfahren zum Betrieb auf bevorzugte Verwendungen der Vorrichtung.

Es ist notorisch bekannt, dass Lebewesen unter dem Einfluss von toxischen Substanzen in ihrer Vitalität beeinträchtigt werden. Dies genügt zum Nachweis des Vorhandenseins derartiger Substanzen, erlaubt aber keine Rückschlüsse auf das Mass der Toxizität und/oder auf die Substanz.

Ein Verfahren zum Nachweis von toxischen Verbindungen in einer Lösung ist aus der DE -A1- 33 45 196 bekannt. Die Bewegung der, zur Lösung zugegebenen Mikroorganismen, wird über die Unterbrechungen in einem Lichtstrahl welcher durch die Lösung führt, mittels einer Photozelle erfasst.

Ein weiteres Verfahren, DE -A1- 29 06 194 betrifft eine Erfassung der Toxizität von Gewässern und Abwässern. Hierfür werden einer Probe auf toxische Verbindungen empfindliche Mikroorganismen zugegebenen. Unterbrechungssignale von Lichtschranken, welche entlang einer Messstrecke angeordnet sind, werden mit einer nicht-toxischen Referenz-Lösung verglichen. Die Unterschreitung einer gewissen Bewegungsaktivität der Organismen führt zu einer entsprechenden Reduktion der Signale, so dass ein Alarm ausgelöst wird.

DE -A1- 39 22 358 beschreibt ein weiteres Verfahren zur Schadstofferkennung in wässrigen Flüssigkeiten. Die Probe, mit zugegebenen Mikroorganismen, wird auf die Objektbühne eines Mikroskops gelegt und mit pulsierendem Licht durchstrahlt. Das durchgelassene Licht wird auf einem photographischen Film registriert. Durch ein Ausmessen zweier benachberten Positionen des selben Mikroorganismus, kann, da die Pulsfrequenz des Lichtes bekannt ist, eine Motilitätsmessung anhand der vom Mikroorganismus pro Zeiteinheit zurückgelegten Wegstrecke erfolgen.

Eine Methode zur Ermittlung von Bioassay-Daten für die Bestimmung der Konzentration von toxischen Substanzen in wässrigen Lösungen ist bekannt (WO 90/12886). Die Daten stammen von biologisch-chemischen Analysen mittels Fluoreszenzmessungen. Diese beruhen auf dem Stoffwechsel von Mikroorganismen, welche eine zugesetzte Substanz stoffwechselbedingt in fluoreszierende Produkte umwandeln. Unter dem Einfluss der toxischen Substanz wird die Stoffwechselaktivität der Mikroorganismen beeinträchtigt.

Im weiteren ist eine Vorrichtung zur Bestimmung der Schwimmgeschwindigkeit von Mikroorganismen mit zwei zu einander synchronisierten Videokameras in REVIEW OF SCIENTIFIC INSTRUMENTS, Bd. 62, Nr. 2, Feb 1991, New York, S. 540-541 beschrieben. Die Bilder der beiden Kameras werden auf einem Videorekorder aufgezeichnet, um ein dreidimensionales Bild zu erzeugen.

Es ist daher Aufgabe der Erfindung eine Vorrichtung und ein Verfahren zu schaffen, welche eine quantitative Aussage über die Toxizität von Substanzen erlauben und zumindest beitragen zur Verminderung von heute noch üblichen, langwierigen Versuchen mit Säugetieren im Rahmen notwendiger Sicherheitsprüfungen.

Zudem soll diese Vorrichtung eine Automatisierung von Überwachungen erlauben, wobei sich die Auswertung rationalisiert und aussagekräftig dokumentieren lässt.

Erfindungsgemäss wird diese Aufgabe nach den Merkmalen des Anspruch 1 gelöst,

Der im Patentanspruch verwendete Begriff "Beweglichkeit" wurde von der Festkörperphysik übernommen und beschreibt die "Bewegungsaktivität" und insbesondere die "Mobilität" der Testorganismen, die in wässrigen Medien am einfachsten als deren "Ortsverschiebung" pro Zeiteinheit beobachtet wird.

Diese Beweglichkeit kann aber auch auf die Beobachtung der Funktion einzelner Organe der Testorganismen angewandt werden.

Eine erhöhte Frequenz der Beweglichkeit einzelner Organe lässt auf eine spezifische Toxizität schliessen, wobei die resultierende "Ortsverschiebung" der Organismen nicht zwingend beeinflusst ist.

Bei das Zellwachstum hemmenden Substanzen liefert die Beobachtung bzw. Messung der Grösse der Testorganismen in Funktion der Zeit ein Mass für diese spezifische Art der Toxizität.

Derartige Toxizitätsbestimmungen lassen sich parallel zur Beweglichkeit, aber auch von dieser getrennt, mit den gleichen apparativen Mitteln ausführen.

Nach der Erfindung lassen sich Kleinlebewesen zur Bestimmung der Toxizität von Substanzen verwenden, indem der Einfluss von einzelnen und/oder von Kombinationen von Substanzen auf die Veränderung an diesen Tieren gemessen wird. Durch solche Messungen lässt sich die notwendige Anzahl Versuche an leidenden Labortieren massiv verringern.

Bevorzugt wird zur Erhöhung der Aussagekraft der Messungen eine Vielzahl von voneinander getrennten Gruppen gleichartiger Testorganismen beobachtet und pro Gruppe gemeinsam ausgewertet.

Ein weiterer Vorteil des Verfahrens liegt in der Einfachheit und raschen Handhabung sowie in seiner Kostengünstigkeit.

Der Begriff der Toxizität ist jedoch nicht auf Giftwirkung im üblichen Sinne beschränkt; er kann auf jede erkennbare Wirkung (Reaktion) auf einen lebenden Organismus ausgedehnt werden.

Die Vorrichtung zur Durchführung des Verfahrens ist dadurch gekennzeichnet, dass eine Bilderfassungseinheit vorgesehen ist, welche die Objekterkennung und eine sequenzielle Aufnahme der Lage des Objekts, in wenigstens einer Ebene, in vorbestimmbaren Zeitintervallen vornimmt, dass der Bilderfassungseinheit eine Auswerteanordnung mit einem Rechner und Darstellungsgeräten nachgeschaltet ist, welche eine statistische Auswertung der Beweglichkeit der Organismen vornehmen, in Relation zur vorhandenen Toxizität.

Im einfachsten Fall wird dabei die Bilderfassungseinheit mechanisch verschiebar ausgestaltet und über die zu untersuchenden Proben geführt.

Als Veränderung der Beweglichkeit wird eine durch den Testorganismus pro Zeitintervall zurückgelegte Wegstrecke gewertet.

Signifikante Werte der Beweglichkeit können durch die in Anspruch 9 spezifizierte Beschleunigung, berechnet aus den Geschwindigkeitswerten, gewonnen werden.

Die gemäss der Erfindung ermittelte Toxizität ist eine relative Toxizität, bezogen auf eine Standard-Toxizität.

Sowohl die Bestimmung der Bewegungsgeschwindigkeit als auch die Berechnung der resultierenden Beschleunigungswerte und/oder von Änderungen in der Grösse der Organismen können in einer Ebene als auch dreidimensional durchgeführt werden, je nach apparativem Aufwand. Daraus lassen sich in einfachster Weise charakteristische Messkurven aufzeichnen, die für die Einflussnahme von Stoffen auch auf höhere Lebewesen interpretierbar sind.

Die Erfindung wird zweckmässigerweise unter Verwendung von niederen Tieren als Testorganismen angewandt. Besonders geeignet sind hierfür Organismen die im Wasser leben, da ihre Beweglichkeit am einfachsten beobachtbar ist.

Die Testdurchführung unter Verwendung von Artemia salina Nauplien ist weder durch jahreszeitliche Abhängigkeit noch durch Fütterung und Aufzucht beeinflusst, wohl aber durch die Umgebungstemperatur und den Faktor Zeit, sodass nur automatisierte Verfahrensabläufe als professionelle Untersuchungsmittel in Frage kommen.

Durch eine Erhöhung der Umgebungstemperatur gegenüber der Normaltemperatur wird erwartungsgemäss eine Zunahme der Beweglichkeit der Testorganismen festgestellt.

Um wiederholbare Resultate zu erreichen ist es empfehlenswert, die Messungen bei 22 °C +/- 1 °C durchzuführen und zu normieren. Dieser Wert lässt sich mit apparativen Mitteln relativ leicht konstant halten und verhindert zudem eine zu hohe Verdunstung bzw. Wasserdampfbildung bei Langzeitbeobachtungen.

Artemia salina bildet unter schlechten Lebensbedingungen Zysten (Eier, die im frühen Gastrulastadium arretiert sind) und so über viele Jahre aufbewahrt werden können.

Dementsprechend ist es sehr zweckmässig die Untersuchungen unter Verwendung von Kiemenfüssern (Euphyllopoda) wie Artemia salina Nauplien (in Embryonal-Stadien 3 und 4 mit einer Grösse von ca. 0,9 - 1,15 mm) in Meerwasser durchzuführen.

Ebenfalls sind freischwimmende Rotatorien (Ploima) einsetzbar.

Für spezielle Untersuchungen, beispielsweise bei der Überprüfung karzinogener Stoffe, sind Wasserflöhe (Cladocera), Anspruch 10, bevorzugt, da sie in einfacher Weise Beobachtungen über zwei Generationen ermöglichen.

Nachdem Artemia salina ein Meerwassertier ist, stellt künstliches Meerwasser eine adäquate Umgebung für die Messung dar, Anspruch 11.

Um eventuelle unkontrollierte Reaktionen zwischen der toxischen Substanz mit Meer- und/oder Leitungswasser auszuschliessen, kann es vorteilhaft sein Süsswasserarten der Kiemenfüsser in künstliches Süsswasser, bestehend aus destilliertem Wasser mit Natriumbicarbonat, einzusetzen. Ebenso empfiehlt sich der Einsatz von künstlich hergestelltem Meerwasser.

Andere Organismen, wie Kaulquappen etc. können erfindungsgemäss ebenfalls verwendet werden.

In einer bevorzugten Ausführungsform des Verfahrens erfolgt die Messung unter Verwendung einer Mikrotiterplatte.

Zur bevorzugten elektrooptischen Instrumentalanalyse werden in die oben beschriebenen Gefässe die zu ermittelnden wasserlöslichen Substanzen in Wasser bzw. einem künstlichen Meerwasser gelöst und der Testorganismus zugesetzt und deren Reaktionen elektronisch ausgewertet.

Es wird dabei die Veränderung der Beweglichkeit der Testorganismen pro Gefäss gemittelt und mit bereits früher erfassten Werten verglichen. Entsprechende Korrelationen erlauben quantitative Aussagen über die vorhandene Toxizität.

Die obigen Untersuchungen sollen zweckmässigerweise immer unter den selben Bedingungen durchgeführt werden, da das Resultat durch die Temperatur beeinflusst wird.

Der Verfahrensablauf lässt sich durch das in Anspruch 13 beschriebene systematische Zupipettieren massgeblich optimieren; potentielle Fehlerquellen lassen sich dadurch weitgehend eliminiern.

Die zur Durchführung des Verfahrens vorgesehene Vorrichtung erlaubt eine einfache Auswertung der beobachteten Bilder durch übliche EDV-Mittel; durch die in Anspruch 2 erwähnte Quotientenbildung aller zurückgelegten Wegstrecken pro Zeitintervall und pro Bild lässt sich eine relative mittlere Geschwindigkeit der Testorganismen bestimmen bzw. durch die zweite Ableitung auch deren Beschleunigungswerte.

Von besonderer Bedeutung ist eine Unterscheidung zwischen bewegten und nicht bewegten Objekten, da sich viele Testorganismen einerseits während der Beobachtungsdauer häuten und diese abgestossenen Häute zu Fehlerfassungen führen können; andererseits müssen die bereits gestorbenen Tiere festgestellt und aus der Rechnung eliminiert werden, Anspruch 3.

Besonders geeignet zur Durchführung des Verfahrens ist eine sogenannte CCD-Shutter-Kamera nach Anspruch 4, da zurzeit nur derartige Kameras in der Lage sind, genügend scharfe und auswertbare Bilder zu liefern.

Durch die in Anspruch 5 vorgeschlagene Anordnung von zwei Kameras lassen sich die Bewegungen der Testorganismen in drei Dimensionen beobachten und vermessen.

Bei einem genügend grossen apparativen Aufwand, dh. bei Verwendung entsprechender Kameras und genügend grosser Bilddatenspeicher und ausreichend schneller Rechner könnten grundsätzlich alle in einer Versuchsanordnung befindlichen Testorganismen gleichzeitig aufgenommen und einzeln ausgewertet werden, vgl. Anspruch 6.

Eine rationelle automatisierte Beobachtung und Auswertung von Testorganismen kann mit den Angaben nach Anspruch 7 oder 8 realisiert werden, vorausgesetzt, dass der verwendete Transportmechanismus erschütterungsfrei arbeitet.

Die in den Ansprüchen 14 - 16 aufgezeigten Anwendungen beziehen sich auf aktuelle Bedürfnisse des Umweltschutzes bzw. der Lebensmitteltechnologie und Pharmaindustrie. Selbstverständlich sind weitere Applikationen denkbar, die auch andere Gebiete der Biologie betreffen.

Die Toxizitätsermittlung, wie sie eingehend mit Artemia salina Nauplien erprobt wurde, ermöglicht die Abklärung der relativen Toxizität von Substanzen in einem wässrigen Medium, dh. von wasserlöslichen Substanzen aber auch von Substanzen, die mit Hilfe von Lösungsvermittlern wie zB. Aceton wasserlöslich gemacht wurden. Sie ermöglicht aber auch - wie in den Ansprüchen 14 - 16 dargestellt ist - einen Aufschluss über die toxische Wirkung von Substanzen auf die Wasserfauna, insbesondere von Insektiziden und Pestiziden.

Im weiteren lassen sich durch periodische Messungen in gleichen Zeitintervallen und mit steigender Konzentration der toxischen Dosen durchgeführte Messungen charakteristische Letaldosis-Kurven aufzeichnen, welche Rückschlüsse auf die Art des toxischen Stoffes zulassen und zur Ergründung des Wirkmechanismus des Stoffes oder der Stoffkombination beitragen.

Nachfolgend werden anhand von Zeichnungen Ausführungsbeispiele der Erfindung näher dikutiert.

Es zeigen:

Fig. 1    eine schematische Darstellung einer elektrooptischen Bilderfassungseinheit mit einem charakteristischen Testorganismus zur Bestimmung von dessen Beweglichkeit,

Fig. 2    eine Gesamtansicht eines Gerätes mit Bilderfassungseinheit und Auswerteanordnung,

Fig. 3    eine Teilschnittdarstellung durch den Geräteschrank Fig. 2, orthogonal zur Frontplatte,

Fig. 4    eine Teilschnittdarstellung durch das Gerät Fig. 2 parallel zur Frontplatte,

Fig. 5    eine Titerplattenanordnung zur Aufnahme von sechs Probenplatten,

Fig. 6    ein Flussdiagramm, welches den Programmablauf zur automatisierten Bestimmung der Beweglichkeit von Testorganismen aufzeigt,

Fig. 7    eine charakteristische mittlere, zeitliche Geschwindigkeitsabnahme von lebenden Testorganismen in drei ausgewählten toxischen Lösungen, beobachtet über einen Zeitraum von 10 Stunden,

Fig. 8    einen Toxizitätstest mit Artemia salina in einer Paraoxon Lösung, wobei in der Abszisse die Konzentration und in der Ordinate der Anteil toter Organismen aufgetragen sind,

Fig. 9    einen weiteren Toxizitätstest, wobei hier die Geschwindigkeit der Testorganismen in Funktion der Konzentration von Paraoxon, zusammen mit einer überlagerten Letalitätsdosiskurve dargestellt ist,

Fig. 10    eine Videoaufnahme einer Gruppe von lebenden Testorganismen in einem wässrigen Medium zu Beginn der Beobachtung,

Fig. 11    eine weitere Videoaufnahme der Gruppe von noch lebenden Testorganismen Fig. 10 nach 24 Stunden, wobei ein Teil des Mediums verdunstet ist,

Fig. 12    ein Aktions/Zeitdiagramm welches die Vorbereitung, Durchführung und Auswertung der Messung der Beweglichkeit von Testorganismen aufzeigt,

Fig. 13    die Messung von bis zu 6 Titerplatten nach dem Diagramm Fig. 12,

Fig. 14    die Messung von bis zu 10 Platten, wobei für eine Platte eine Zeitdauer von bis zu 10 Minuten zugelassen ist,

Fig. 15    sequentiell ablaufende Einzelmessungen, dargestellt im Zeitintervall der Messung einer Platte,

Fig. 16    die zum Messen eines einzelnen Behälters vorgesehenen Intervalle, wobei die Zeiträume zwischen den Einzelmessungen durch den Transportmechanismus der Kamera bestimmt sind und

Fig. 17     eine Sequenzauswertung aller Abläufe die notwendig sind, um ein einzelnes Gefäss in einer Titerplatte auswertbar zu registrieren.

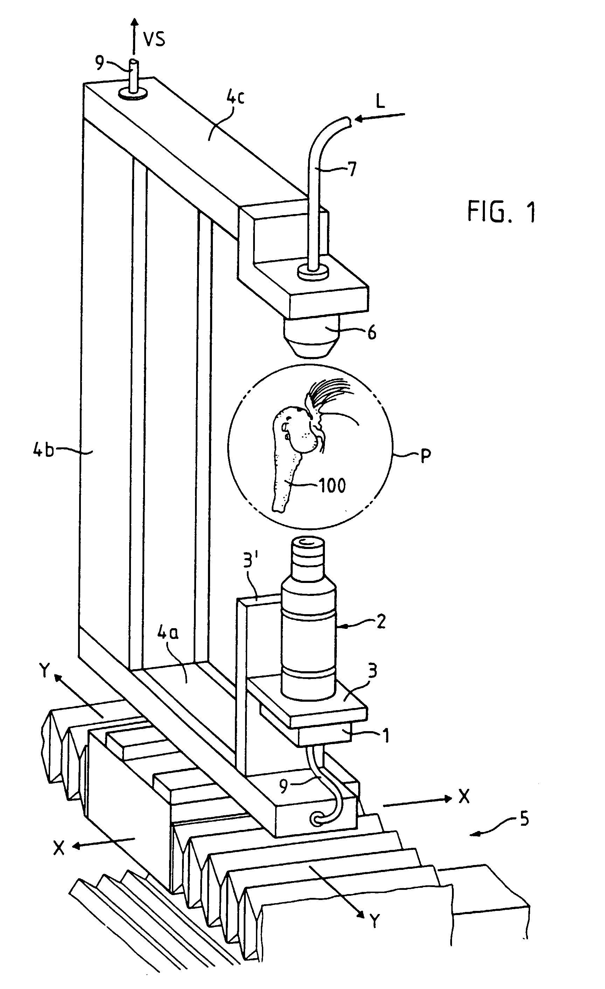

In Figur 1 ist mit 1 eine Videokamera (CCD-Shutter-Kamera; Firma Sony Typ XC-77RR-CE) bezeichnet. Die auf die elektronische Kamera 1 aufgesetzte Optik 2 ist ein Makro-Objektiv (Sony Typ CM50). Die Videokamera 1 ist auf zwei Supports 3,3' in an sich bekannter Weise vertikal an einem C-Arm 4a - 4c angeordnet. Der C-Arm ruht auf einer handelsüblichen Positionierungs-Einheit 5, ein XY-Kreuztisch mit zwei Vorschubeinheiten (Firma ISEL Automation D-6419 Eiterfeld) mit entsprechenden Schrittmotoren, welche eine Verschiebungslänge von 400 mm in X-Richtung bzw. 300 mm in Y-Richtung bewirken.

Zwischen der Optik 2 und dem Lichtverteiler 6 befinden sich Proben P, charakterisiert durch eine Artemia salina 100, welche im Durchlichtverfahren beobachtet wird.

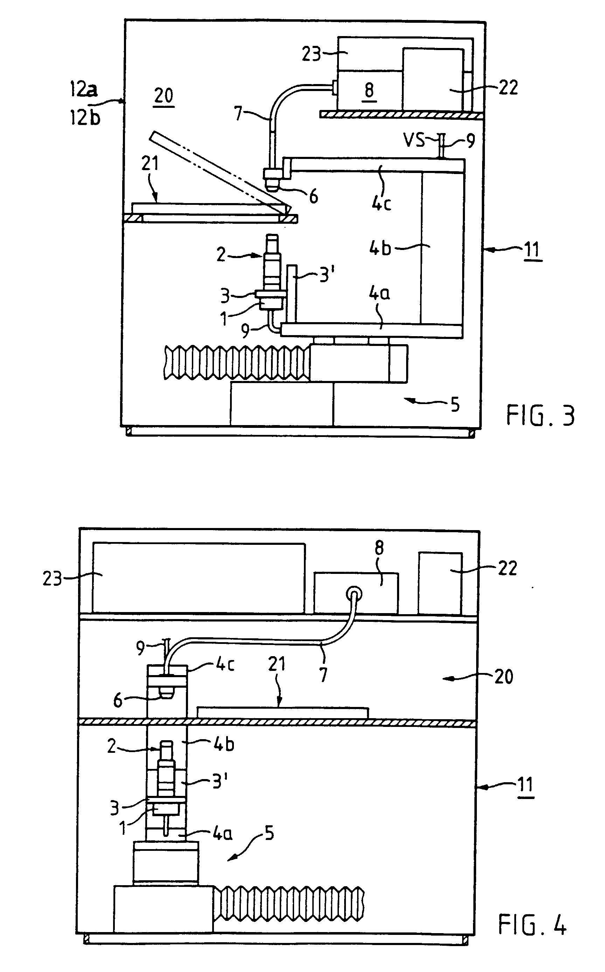

Vertikal oberhalb der Optik 2 der Kamera 1 ist im C-Arm ein Lichtverteiler 6, angeordnet, welche über einen Lichtleiter 7 von einer hier nicht dargestellten Lichtquelle gespeist ist. Die Videosignale VS werden über eine Signalleitung 9 nach oben geführt, vgl. Fig. 3, Fig. 4.

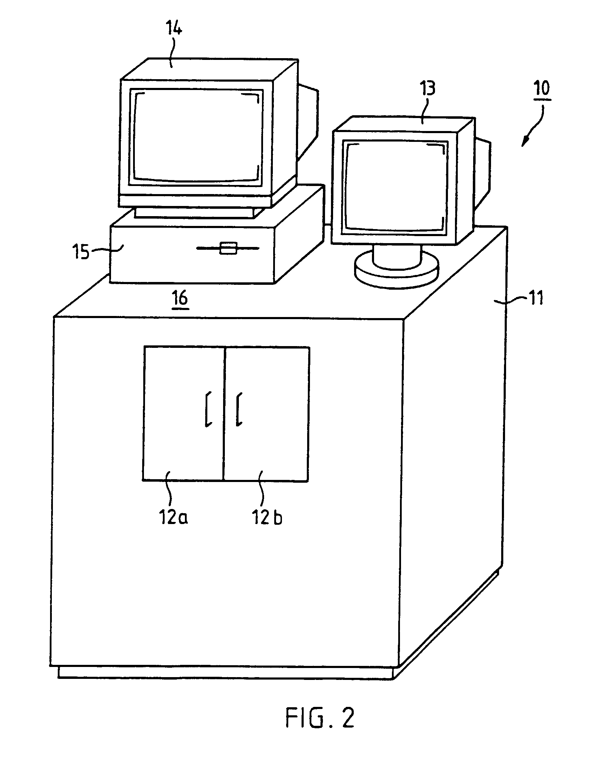

Die gesamte Vorrichtung zur Beobachtung und Auswertung von Testorganismen ist in einer Bilderfassungs- und Auswerteeinheit 10, 10', in Fig. 2, mit 10 bezeichnet, zusammengefasst. In einem Geräteschrank 11 kann über zwei Türen 12a, 12b ein Probenraum mit Experimenten beschickt werden. Auf dem Geräteschrank 11 befindet sich eine handelsübliche Zentraleinheit 15 eines Personal Computers (PC) mit einem ebenfalls üblichen Mcnitor 14. Neben dem PC ist, auf einem drehbaren Ständer angeordnet, ein weiterer Monitor 13, welcher die Aufnahmen der im Geräteschrank 11 befindlichen Video-Kamera darstellt. Auf dem Geräteschrank 11 ist eine Ablagefläche 16 für hier nicht gezeichnete, aber für die Bedienung des Gerätes erforderliche Bedienungs- und Eingabegeräte (Tastatur, Maus etc.) vorgesehen.

Die Schnittdarstellung Fig. 3 zeigt das Innere des Geräteschrankes, wobei die Orientierung des Schnittes durch die Lage der Türen 12a, 12b, vgl. Fig. 2, gegeben ist. Hier sind wiederum der C-Arm 4a, 4b, 4c mit seinen Bestandteilen, Fig. 1 ersichtlich; ebenso die Positionierungseinheit 5 sowie der Lichtleiter 7, welcher mit einer üblichen Lichtquelle 8 verbunden ist.

In einem durch die Türen 12a, 12b zugänglichen Probenraum 20 befindet sich eine Titerplattenanordnung 21, welche Proben mit Testorganismen aufnimmt. Versorgt ist das Ganze durch eine handelsübliche, unterbrechungsfreie Stromversorgung 22, Fig. 3 und 4. Sämtliche für die Steuerung der Positionierungseinheit 5 notwendigen Signale werden in einer Steuerung 23 generiert. Die Videosignale VS werden zu einer üblichen Kamera-Kontrolleinrichtung im Steuer-Computer 15 geführt.

Nicht dargestellt sind die notorisch bekannten Peripheriegeräte wie Drucker, Plotter etc., welche an den PC anschliessbar sind.

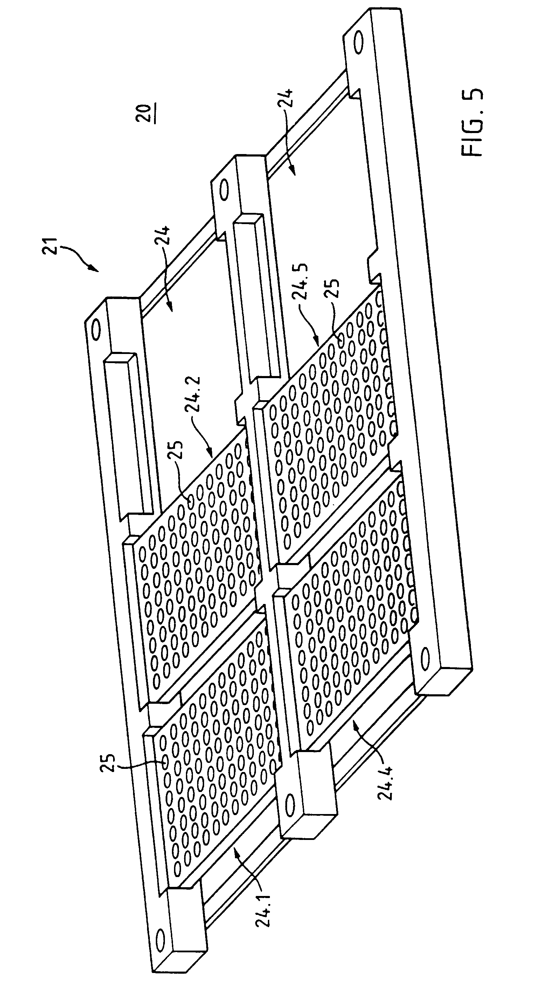

In Figur 5 ist die im Probenraum 20 angeordnete Titerplattenanordnung 21 mit ihren wesentlichen Bestandteilen gezeichnet.

Die Anordnung 21 besteht aus einem im Geräteschrank 11 festgeschraubten Rahmen mit Öffnungen 24, in welchen sechs Mikrotiterplatten 24.1 - 24.6 mit je 96 Gefässen einsetzbar sind und welche pro Gefäss je eine Probe mit Testorganismen (zirka 12 Artemia salina) enthalten. Ein nicht dargestellter Schutzdeckel verhindert die Störung der Proben durch Staubeinwirkung und/oder Wärmestrahlungen etc.; ebenso sind die einzelnen Titerplatten zur Reduktion der Verdunstungswirkung mit einem transparenten Kunststoff abgedeckt.

Mit der vorgängig beschriebenen Vorrichtung lässt sich die Beweglichkeit von Testorganismen messen. Am einfachsten geschieht dies durch die Feststellung der Fortbewegungsgeschwindigkeit der Organismen in einer Testlösung, wobei die relative Geschwindigkeit auf eine Kontroll-Lösung (ohne toxische Substanz) bezogen ist.

Der Benutzer wird beim Entwerfen des Experiments zweckmässigerweise über ein PC-Programm angeleitet. Dieses erfragt über die Anzahl Konzentrationen (in Mol oder Prozent) und Parallelbestimmungen, Leerwerte, Kontrollen und deren Anordnung, sowie über die Plazierung des Experiments in den jeweiligen Gefässen der Mehrgefässanordnung. Im weiteren verlangt das Programm Angaben über den Namen der Testsubstanz, deren Molekulargewicht, höchste zu testende Konzentration und Wahl der linearen oder logarithmischen Verdünnungsreihe. Nach Eingabe des Totalvolumens für den Test, des Volumens in dem die Testorganismen (beispielsweise Artemia salina) sowie die Testsubstanz zugesetzt werden, berechnet das Programm die für das Experiment benötigte Menge der Testsubstanz. Nach der Erfassung der abgewogenen Menge können nun direkt unter Führung des Programms, die benötigten Verdünnungen hergestellt werden. Durch eine optische Hilfe auf dem Bildschirm, wird das Zupipettieren erleichtert.

Eine bevorzugte Meerwasserlösung enthält 2800 mg NaCl, 342 mg $MgSO_4 \cdot 7H_2O$, 234 mg $MgCl_2 \cdot 6H_2O$, 122 mg $CaCl_2 \cdot 2H_2O$, 20 mg $NaHCO_3$, 74 mg KCl auf 100 ml destilliertes Wasser.

Durch eine elektrooptische Bestimmung wird in einem ersten Schritt die Anzahl der Testorganismen bestimmt, welche sich in der mit der zu untersuchenden Substanz versetzten, künstlichen Meerwasserlösung befinden.

Die Beobachtung des Einflusses der Substanz auf den Organismus erfolgt über das dargestellte Bildanalysensystem zu vorgewählten Zeitpunkten. Sie gestattet folgende Kriterien zu erfassen:

-     Anzahl der Organismen

- Bestimmung, wie viele davon nicht mehr aktiv sind

- Ermittlung der durchschnittlichen Beweglichkeit bzw. relativen Bewegungsgeschwindigkeit

- Durchschnittliche Grösse (relative Fläche) der Organismen; Berechnung ihres individuellen, lageabhängigen Flächenschwerpunktes.

Die elektrooptische Bestimmung wird grundsätzlich wie folgt durchgeführt:

Die Proben werden in der Mikrotiterplatte, die den Test enthält, im beschriebenen Analysenautomaten plaziert. Zu den vorgewählten Messzeiten fährt eine elektronische Kamera unter die einzelnen Messplätze der Mikrotiterplatte und nimmt 12 bis 36 Bilder auf. Gleichzeitig wird die jeweilige Probe von oben beleuchtet. Damit diese Bilder scharf ausfallen, muss mit einer Kamera mit Verschlusszeitsteuerung gearbeitet werden. Das Objektiv dieser Kamera ist so ausgelegt, dass einerseits das Bild formatfüllend aufgenommen wird; anderseits die ganze Tiefe der Flüssigkeit (ca. 1 cm) scharf abgebildet werden kann. Die Beleuchtung erfolgt über einen Lichtleiter, der an den C-Arm zusammen mit der Kamera befestigt parallel fährt. Die Auflösung der Bilder wurde so optimiert, dass mit einem Minimum an Bildpunkten die ganze Analyse durchgeführt werden kann. Dies erlaubt, die Rechenzeit für die Bildanalyse möglichst tief zu halten. Es wurde eine Auflösung von 256 mal 256 Bildpunkte gewählt. Diese Auflösung gestattet, die Umrisse der Organismen noch eindeutig zu erkennen. Dabei wird auch eine Mess- und Auswertezeit von ungefähr drei Minuten pro Mikrotiterplatte (mit 96 Gefassen), dh. ungefähr 2 Sekunden pro Messplatz erreicht.

Die Daten werden nach Abschluss der Messung auf einer Diskette gespeichert und können nun direkt mit einem PC-Programm ausgewertet werden. Dabei werden die Daten zusätzlich grafisch dargestellt, und die charakteristischen Toxizitätswerte direkt ermittelt. Diese Daten werden nun in einer Datenbank so abgespeichert, dass sie jederzeit zu Vergleichszwecken zur Verfügung stehen.

Zur Durchführung der obigen Instrumental-Analyse von flüssigen Proben, werden Mikrotiterplatten verwendet, die aus transparentem Material bestehen. Durch eine, aus zwei Materialien unterschiedlicher physikalischer Eigenschaften zusammengefasste Mehrgefässanordnung, lässt sich die Grenzflächenspannung an der Flüssigkeitsoberfläche einstellen, so dass diese wenigstens annähernd planar ist. Bevorzugterweise werden dabei die Böden der Mehrgefässanordnung aus transparentem Material (CH-Gesuch Nr. 03 141/93-6), beispielsweise Polystyrol oder einem Copolymeren eines Polystyrols gefertigt und die Wände aus wenig lichtdurchlässigem Material, beispielsweise Polyolefinen oder Polytetrafluorethylen, die keine Meniskusbildung oder Proteinadsorption hervorrufen.

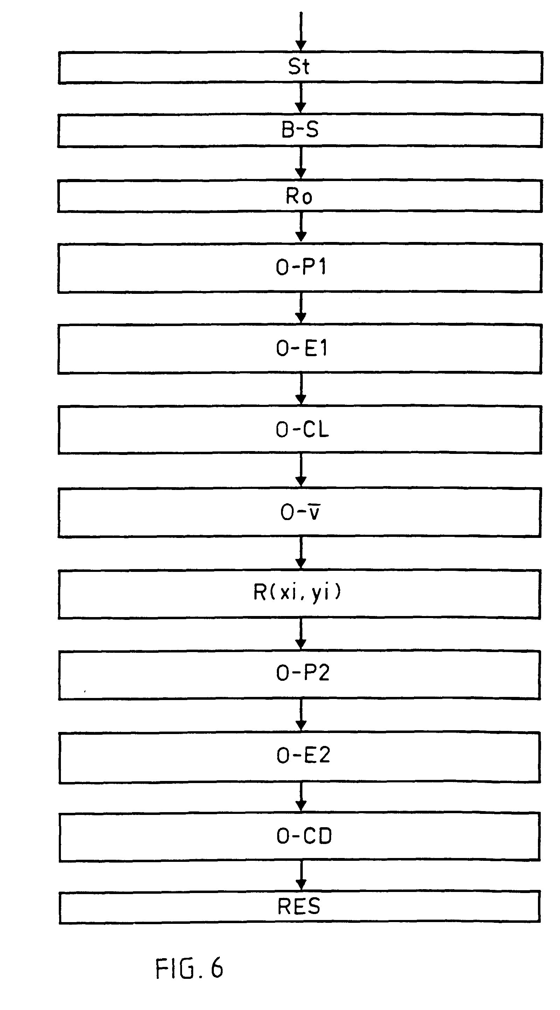

Der oben skizzierte, realisierte Programmablauf in der Vorrichtung gemäss Fig. 1 bis 5 ist aus der Darstellung Fig. 6 zu entnehmen, wobei der Start mit St und das Programmende mit RES = Resultat bezeichnet sind.

Nach dem Start erfolgt eine Aufnahme der Bildsequenzen mit konstantem Zeitintervall At, und zwar online, wobei die Werte in bekannter Weise binärisiert und zwischengespeichert werden: Mit B-S bezeichnet.

Anschliessend erfolgt eine Bestimmung des Messfensterradius, abhängig vom Flüssigkeitsstand: Ro bezeichnet.

In einem nächsten Schritt erfolgt eine Objektbezeichnung und eine Bestimmung der Objektparameter bei allen Bildern innerhalb des Messfensters, dh. die Schwerpunktkoordinaten, die relative Fläche, der Umfang und die Anzahl visuell (elektronisch) feststellbaren Löcher im Objekt werden zur Identifikation, in jedem Gefäss vermessen: O-P1.

Danach werden unzulässige Objekte bei allen Bildern eliminiert, wobei das Kriterium durch zulässige Vorgaben von Fläche und Umfang programmiert ist: O-E1.

Jetzt wird die Anzahl Testorganismen pro Sequenz zu allen Bilddaten ermittelt, mit O-CL = Objekt-Auszählung bezeichnet; dies entspricht der Gesamtzahl lebender Organismen pro Bild.

Durch eine Messung der Geschwindigkeit der Organismen aus allen vorgängig ermittelten Bilddaten wird ein Mittelwert gebildet. Dieser Wert ist in Fig. 6 mit O-v = mittlere Geschwindigkeit der Organismen bezeichnet, wobei als Messreferenz jeweils die momentane Koordinate des Flächenschwerpunktes in der Silhoutte des Organismus dient.

Anschliessend wird ein Resultatbild auf der Basis von Bildpunkten mit deren Koordinaten xi,yi erzeugt und derart normiert, dass die Zahl 1 ein nicht bewegtes, totes Objekt bedeutet. Alle übrigen Bildpunkte xi, yi werden dabei Null (= 0) gesetzt. Bezeichnet ist diese Verfahrensstufe mit R(xi,yi).

Unter O-P2 erfolgt wiederum eine Objektbezeichnung und eine Bestimmung der Objektparameter bei allen Bildern innerhalb des Messfensters, dh. die Schwerpunktkoordinaten, die relative Fläche, der Umfang und die Anzahl Objekte im Gefäss werden vermessen.

Danach werden wiederum die unzulässigen Objekte bei allen Bildern eliminiert, wobei das Kriterium durch zulässige Vorgabe von Fläche und Umfang programmiert ist: O-E2.

Danach werden die verbleibenden, toten Objekte im Resultatbild gezählt: O-CD.

Unter RES erfolgt die Auswertung der Ergebnisse mit statistischen Methoden in an sich bekannter Weise (Probit etc.)

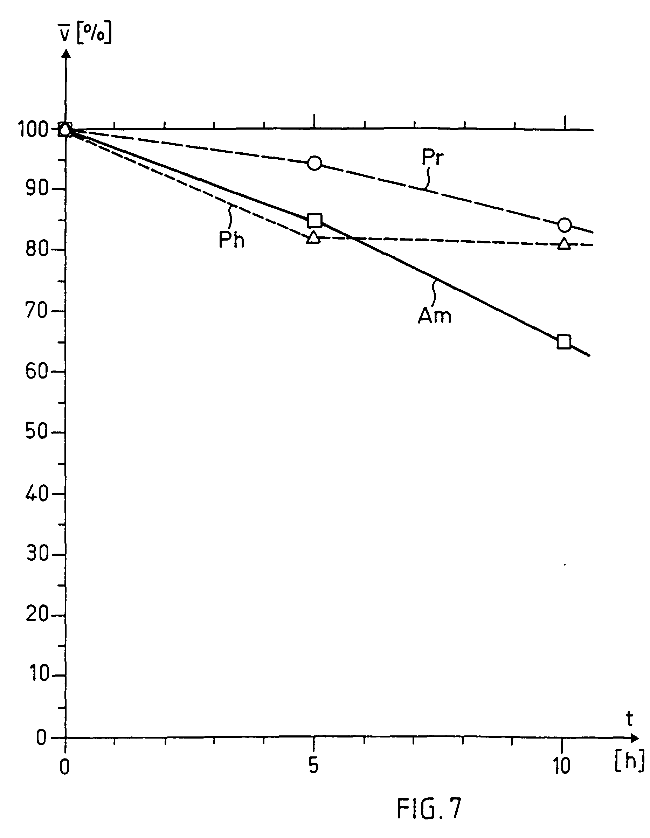

Wie Fig. 7 zeigt, wurden in drei bekannten, ausgewählten toxischen Lösungen, enthaltend Phenobarbital mit Ph bezeichnet, Amphetaminsulfat mit Am und D-Propoxyphen mit Pr bezeichnet. Die Ortsverschiebungen pro Zeiteinheit von Artemia salina wurde als mittlere

Geschwindigkeit $\bar{v}$ ermittelt und aufgezeichnet.

In einer Kontroll-Lösung, dh. in einer nachfolgend beschriebenen Lösung aus künstlichem Meerwasser, wurden typische mittlere Geschwindigkeiten der Artemia salina von $5 \cdot 10^{-3}$ m/s bis $6 \cdot 10^{-3}$ m/s festgestellt.

Wie aus der Darstellung Fig. 7 zu entnehmen ist, erfolgt im Falle von D-Propoxyphen eine relativ lineare Geschwindigkeitsabnahme über einen Zeitraum von 10 Stunden auf zirka 84 % der ursprünglichen Geschwindigkeit. Bei Amphetaminsulfat resultiert eine Abnahme der Geschwindigkeit auf zirka 65 % der ursprünglichen Geschwindigkeit und bei Phenobarbital auf zirka 80 %.

Es hat sich gezeigt, dass je nach Toxizitätsäquivalentfaktor (Art oder Gruppe oder Klasse des toxischen Stoffes) charakteristische Geschwindigkeitsabnahmen feststellbar sind. Dies kann auf signifikante und damit der Erkennung dienende Wirkungen einzelner Stoffe und Kombinationen von Stoffen ausgedehnt werden.

Je nach Art der Wirkung des Stoffes auf den Organismus kann durch eine entsprechende Bildauswertung die Aussagekraft der Messung durch eine Anpassung des Messverfahrens optimiert werden. Beispielsweise kann anstelle der Beobachtung der Fortbewegungsgeschwindigkeit die spezifische Beweglichkeit einzelner Organe des Organismus beobachtet werden; ausserdem können zyklische Bewegungen durch die Bildung der ersten Ableitung aus der Geschwindigkeitsmessung hervorgehoben bzw. einfacher erfasst und analysiert werden.

**Praktisches Beispiel zur Bestimmung der Toxizität von Paraoxon:**

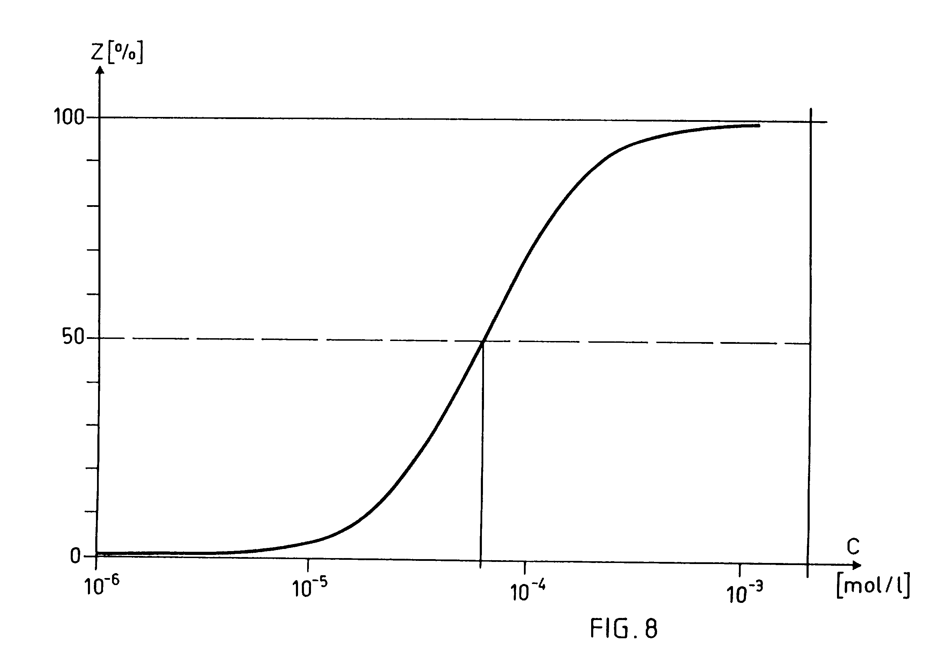

In Fig. 8 ist der Anteil Z an toten Organismen, bezogen auf 100 % zu Beginn der Messung; bei zunehmender Konzentration C von Paraoxon wird ein Ansteigen von Z festgestellt. Diese Art der Auswertung ermöglicht die notorisch bekannte Bestimmung des $LC_{50}$-Wertes der toxischen Substanz.

Die Anzahl der toten Artemia salina wird aufgrund der Messung der Geschwindigkeit der Testorganismen nach dem oben beschriebenen Verfahren berechnet, wobei eine festgestellte Geschwindigkeit Null ein totes Tier bedeutet.

Die in Fig. 8 dargestellte Kurve ist ein Mittelwert von vier unabhängigen Versuchsreihen über einen Konzentrationsbereich von Paraoxon in künstlichem Meereswasser über drei Dekaden. Der Mittelwert der so gemessenen $LC_{50}$-Werte bei einer Temperatur von 22°C beträgt $5,9 \cdot 10^{-5}$ Mol/l bei einer maximalen Streuung von 33 %.

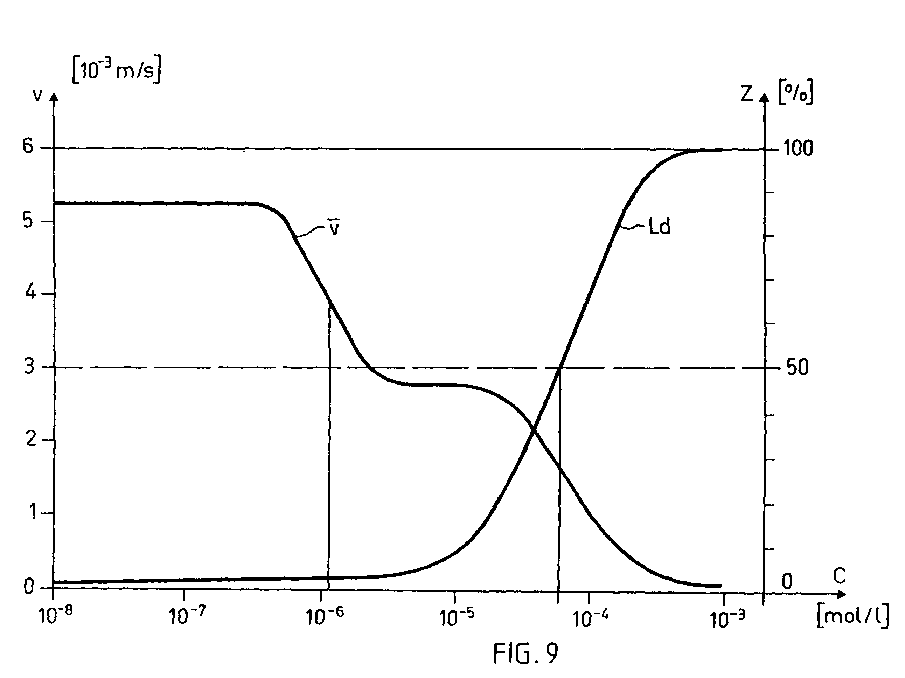

Fig. 9 zeigt die Geschwindigkeit $\bar{v}$ der Artemia salina Testorganismen in einer Versuchsreihe gemessen 30 min. nach Zugabe der Paraoxon-Lösungen mit steigenden Konzentrationen. Es sind hier zwei drastische Abnahmen der Geschwindigkeit ersichtlich. Die zweite Abnahme, bei zirka $1 \cdot 10^{-4}$ mol/l Paraoxon entspricht dem $LC_{50}$-Wert, welcher aus der überlagerten Letalitätsdosiskurve entnehmbar ist.

Die signifikante erste Geschwindigkeitsabnahme liegt bei etwa $1 \cdot 10^{-6}$ mol/l Paraoxon und entspricht der "Effect Concentration" $EC_{50}$. Diese ermittelt eine Indikation der Funktion der toxischen Substanz.

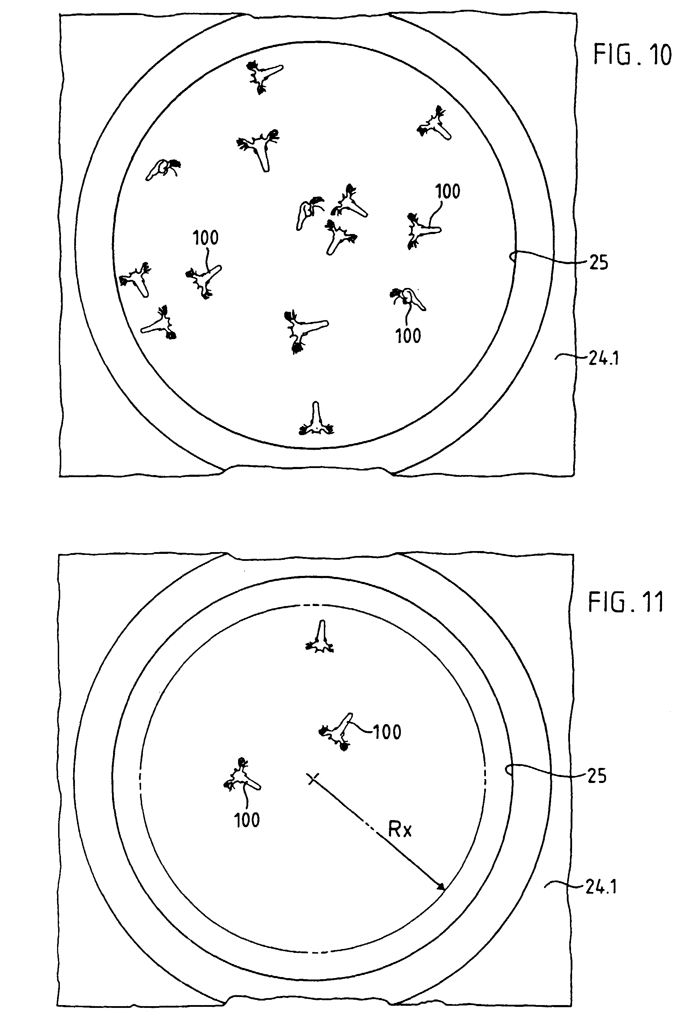

Charakteristische Videoaufnahmen sind aus Fig. 10 (zu Beginn des Experimentes) und Fig. 11 (Ende des Experimentes nach 24 Stunden) ersichtlich. In der zweiten Aufnahme sind die toten, dh. unbewegten Tiere, in an sich bekannter Weise elektronisch unterdrückt und daher auf dem Monitor 13, Fig. 2, unsichtbar.

In Fig. 11 ist zusätzlich ein Radius Rx eingetragen welcher ein eventuelles Verdunsten von Flüssigkeit mit daraus resultierender Reduktion des Messfenster-Radius andeutet; gestrichelt eingezeichnet.

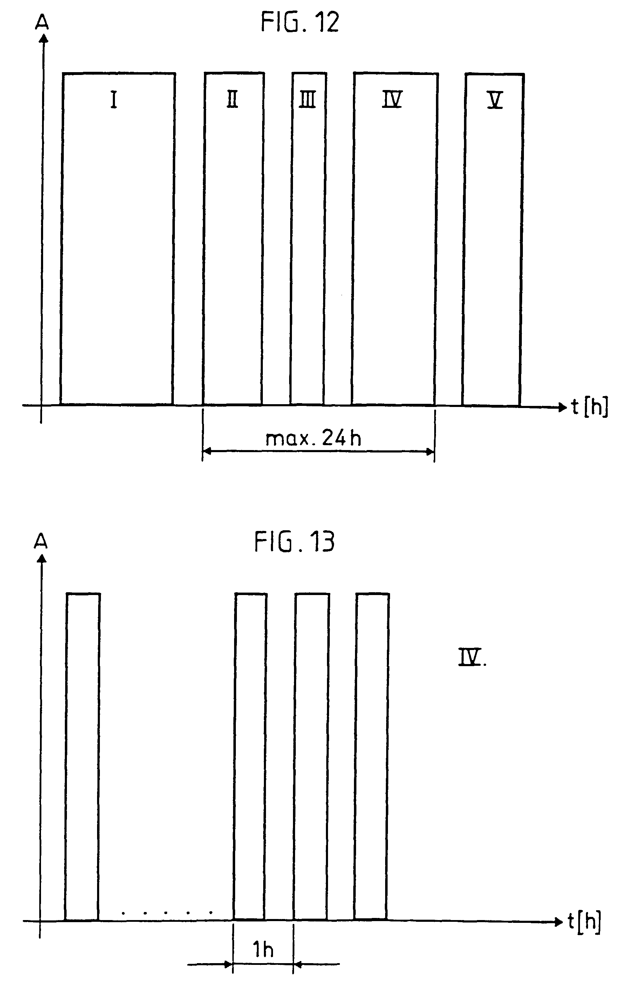

Weitere Einzelheiten im technischen Verfahrensablauf sind den Fig. 12 - 17 zu entnehmen, wobei das Aktions/Zeitdiagramm Fig. 12 den gesamten Verfahrensablauf zeigt. Bezeichnet ist dieses Diagramm in den einzelnen Stufen mit I - V, wobei die Verfahrensschritte auf der Abszisse in der Grössenordnung ihrer Zeitdauer herauslesbar sind.

Dabei bedeuten:

I       die Vorbereitung der Messreihe mit einer "Setup-Hilfe" am PC zum Beschicken der Titerplatten,

II      eine Kalibrierung der Startwerte des Überwachungsprogramms,

III     das Zuführen der toxischen Substanz,

IV      die Aufnahme der Messreihe mit dem Überwachungsprogramm,

V       die Auswertung der Messreihe mit statistischen Analysemethoden und Korrelationen zu bisherigen Messungen (Datenbank).

Die nachfolgenden Fig. 13 bis Fig. 17 betreffen all die Verfahrensstufe IV, was in aus Übersichtlichkeitsgründen in jedem Aktions/Zeitdiagramm vermerkt ist.

Figur 13 zeigt den typischen Ablauf bei der Aufnahme einer Messreihe mit bis zu sechs Titerplatten, wobei im "Stundentakt" während 24 Stunden gemessen wird. Diese Messreihe entspricht in Fig. 12 der Position IV.

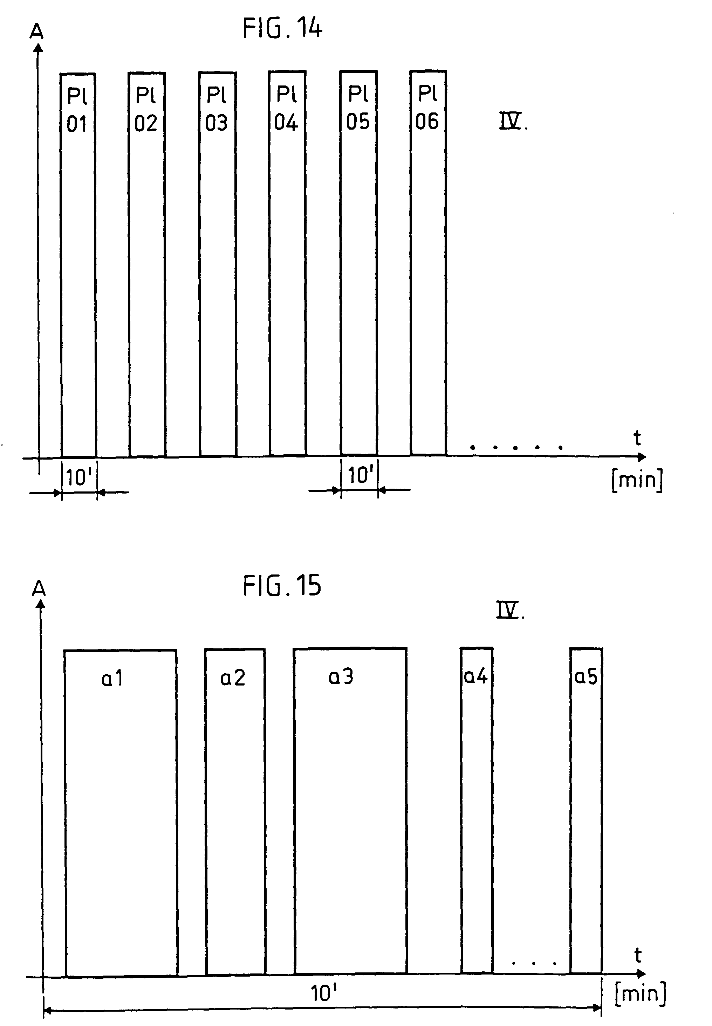

In Fig. 14 ist die Position IV (Aufnahme der Messreihe) im einzelnen dargestellt. Die einzelnen Titerplatten sind hier mit PI 01 - PI 06 bezeichnet; die maximale Messdauer ist in praxi auf 10 Minuten begrenzt.

Aus Fig. 15 lassen sich die einzelnen Verfahrensschritte während der Messdauer herleiten. Dabei bedeuten:

a1      die Erstellung einer Binärisierungsschwelle zur Bildverarbeitung,

a2      die Einstellung bzw. Überprüfung der Fokussierung der Kamera mittels der Bildverarbeitung,

a3        die Prüfung der Orientierung der Titerplatten (zur Festlegung und Berücksichtigung der abnehmenden Konzentrationswerte der Proben),

a4        das Vermessen eines ersten Gefässes,

a5 - a7        das Vermessen eines beliebigen bzw. des n-ten Gefässes.

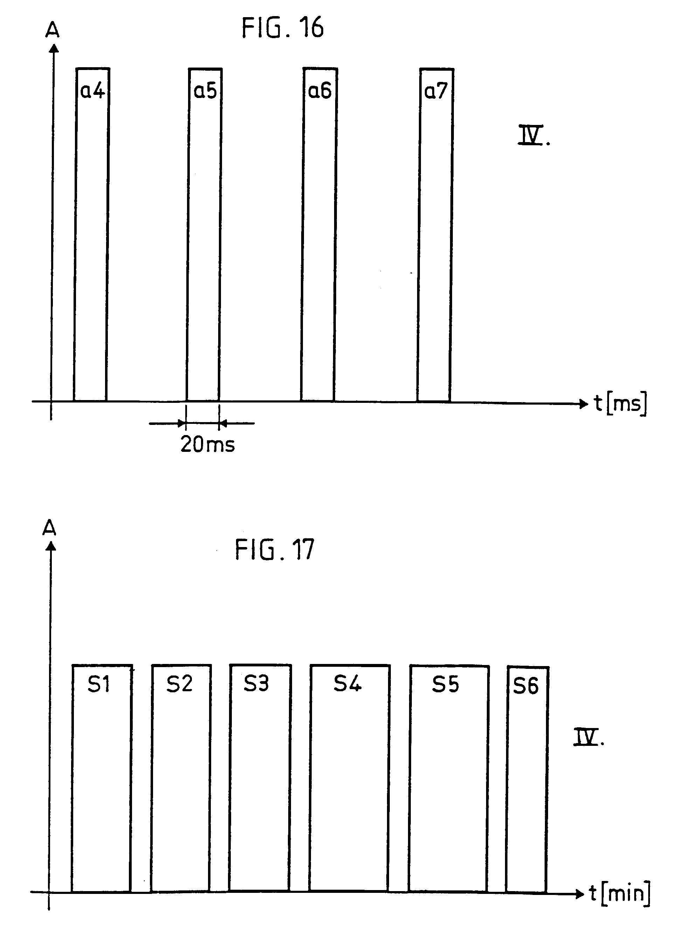

Die Darstellung Fig. 16 zeigt die sequentielle Bildaufnahme pro Gefäss, wobei hierfür in praxi eine maximale Zeitdauer von 20 ms vorgesehen ist.

Fig. 17 zeigt die Sequenzauswertung und zwar sind mit S1 der auswertbare Radius des Gefässes, mit S2 die Schwerpunkts-Koordinaten sämtlicher Testorganismen (im Bild 1) und die Schwerpunkts-Koordinaten sämtlicher Organismen in Bild n bezeichnet, welche durch die Bildverarbeitung bestimmt werden.

In der Folge werden, mit S4 bezeichnet, die Anzahl der Organismen und deren Mittlere Geschwindigkeit aus allen Bilddaten rechnerisch ermittelt.

Die weiteren Sequenzen sind S5, das Kombinieren sämtlicher Bilder zu einem Resultatbild, welches nur tote Organismen enthält und die Sequenz S6, das Auszählen der toten Organismen mittels der Bildverarbeitung.

Die weitgehend anhand von toxischen Stoffen geführten Untersuchungen lassen sich generell auf die Bestimmung von Schwellenwerten, Schwellenreizen, Schwellendosen und Konzentrationen ausdehnen.

Die Verwendung von Kleinlebewesen ermöglicht überdies eine gewaltige Einsparung an Versuchstieren, da deren Zahl durch vorherige Bestimmung der zu testenden Konzentrationen eingeschränkt werden kann.

Denkbar ist eine Anwendung des Erfindungsgegenstandes auf weitere, bisher noch nicht erprobte Organismen wie Nematoda (Fadenwürmer) und andere, wie auch im Mikrobereich liegende Organismen.

Ebenfalls kann das Verfahren mit konventionellen Analysemethoden kombiniert werden, so dass die Messdauer verkürzt und die Aussagekraft der Messung zusätzlich erhärtbar ist, ohne dass aufwendige Versuchreihen erforderlich sind.

**Patentansprüche**

1.    Vorrichtung zur Bestimmung der Toxizität von wasserlöslichen bzw. mit Wasser mischbaren Substanzen, wobei wenigstens eine Referenzmessung und wenigstens eine weitere Messung nach der Zugabe der toxischen Substanzen erfolgen und senkrecht zur Oberfläche des wässrigen Mediums die Bewegunsaktivität von Testorganismen elektrooptisch beobachtet wird, dadurch gekennzeichnet, dass eine Bilderfassungseinheit (10) eine Objekterkennung und eine sequenzielle Aufnahme der Lage des Objekts (100), in vorbestimmbaren Zeitintervallen vornimmt, dass der Bilderfassungseinheit (10) eine Auswerteanordnung (10') mit einem Rechner und Darstellungsgeräten nachgeschaltet ist, welche eine statistische Auswertung der Beweglichkeit und/oder Grösse der Organismen vornehmen, in Relation zur vorhandenen Toxizität.

2.    Vorrichtung nach Anspruch 1, dadurch gekennzeichnet, dass die Bilderfassungseinheit (10) von wenigstens einer, unter den gleichen Bedingungen befindlichen, Gruppe von Testorganismen (100) ein Grauwertbild aufnimmt und dass die nachgeschaltete Auswerteanordnung (10') daraus binäre Schwarz/Weiss-Werte bestimmt und eine sequenzielle Einzelbildauswertung mit einer Angabe der momentanen Koordinaten-Lage der Testorganismen vornimmt, welche im Rechner durch einen Vergleich mit einem zuvor aufgenommenen Bild und wenigstens einer Quotientenbildung mit dem Zeitintervall zwischen den beiden Bildern, ein Relativmass der Beweglichkeit und/oder Grösse der Organismen berechnet, sowie deren Anzahl erfasst.

3.    Vorrichtung nach Anspruch 1, dadurch gekennzeichnet, dass die Auswerteanordnung (10') erfasste Objekte mit der relativen Beweglichkeit Null einerseits im Bild markiert und andererseits diese im Rechner eliminiert.

4.    Vorrichtung nach Anspruch 1, dadurch gekennzeichnet, dass die Bilderfassungseinheit (10) eine einzige CCD-Shutter-Kamera aufweist, welche taktweise über die jeweilige Gruppe von Testobjekten (100) unter den gleichen Bedingungen geführt ist.

5.    Vorrichtung nach Anspruch 1, dadurch gekennzeichnet, dass die Bilderfassungseinheit (10) zwei CCD-Shutter-Kameras aufweist, deren optische Achsen zueinander wenigstens in einem spitzen Winkel angeordnet sind und die Bewegung und/oder Grösse der Testorganismen in drei Dimensionen erfassen.

6.    Vorrichtung nach Anspruch 1, dadurch gekennzeichnet, dass die Bilderfassungseinheit (10) stationär ist und dass diese mehrere, unter unterschiedlichen Bedingungen befindliche Gruppen von Testorganismen (100) gleichzeitig erfasst und der Auswertung zuführt.

7.    Vorrichtung nach Anspruch 1, dadurch gekennzeichnet, dass die Bilderfassungseinheit (10) stationär ist und dass mehrere, unter unterschiedlichen Bedingungen befindliche Gruppen von Testorganismen (100) mit kontinuierlicher Geschwindigkeit an dieser vorbeigeführt sind und dass die Bilderfassungseinheit (10) diese Gruppen sequenziell erfasst und der Auswertung zuführt.

8. Vorrichtung nach Anspruch 1, dadurch gekennzeichnet, dass unter unterschiedlichen Bedingungen befindliche Gruppen von Testorganismen (100) kreisförmig unter der Bilderfassungseinheit geführt sind.

9. Verfahren zum Betrieb einer Vorrichtung nach wenigstens einem der Ansprüche 1 bis 8, dadurch gekennzeichnet, dass als Veränderung der Beweglichkeit die pro Zeitintervall festgestellte Änderung der Geschwindigkeit des Testorganismus gewertet wird.

10. Verfahren zum Betrieb einer Vorrichtung nach wenigstens einem der Ansprüche 1 bis 8, dadurch gekennzeichnet, dass als Testorganismus Wasserflöhe wie Daphnia magna oder Daphnia pulex eingesetzt werden.

11. Verfahren zum Betrieb einer Vorrichtung nach wenigstens einem der Ansprüche 1 bis 8, dadurch gekennzeichnet, dass als wässriges Medium künstliches Meer- oder Süsswasser eingesetzt wird.

12. Verfahren zum Betrieb einer Vorrichtung nach wenigstens einem der Ansprüche 1 bis 8, dadurch gekennzeichnet, dass die Veränderung der Beweglichkeit und/oder Grösse des Testorganismus in einer Mikrotiterplatte beobachtet wird.

13. Verfahren zum Betrieb einer Vorrichtung nach wenigstens einem der Ansprüche 1 bis 8, dadurch gekennzeichnet, dass die Zugabe der toxischen Substanzen durch eine, über einen Bildschirm kontrollierte, Zupipettierung vorgenommen wird.

14. Verwendung der Vorrichtung nach einem der Ansprüche 1 bis 8 zur Bestimmung von toxischen Wirkungen auf die Wasserfauna.

15. Verwendung der Vorrichtung nach einem der Ansprüche 1 bis 8 zur Bestimmung des Einflusses von toxischen Substanzen wie Insektizide und Pestizide auf die Ökologie.

16. Verwendung der Vorrichtung nach einem der Ansprüche 1 bis 8 zur Bestimmung von Nebenwirkungen und Kontrainidikationen von Pharmazeutika und Nahrungsmitteln.

**Claims**

1. A device for determining the toxicity of water-soluble or water-miscible substances, wherein at least one reference measurement and, after the addition of the toxic substances, at least one further measurement is taken and the displacement activity of test organisms perpendicularly to the surface of the aqueous medium is electro-optically observed, characterised in that an imaging unit (10) carries out object identification and sequential recording of the position of the object (100) at predeterminable time intervals, and in that an evaluation system (10') is connected downstream of the imaging unit (10) and comprises a computer and display apparatus which carry out statistical evaluation of the mobility and/or size of the organisms in relation to the existing toxicity.

2. A device according to claim 1, characterised in that the imaging unit (10) records a grey-value image of at least one group of test organisms (100) under the same conditions, and in that the evaluation system (10'), connected downstream, determines binary black/white values therefrom and carries out sequential single-image evaluation with indication of the current co-ordinate position of the test organisms, which, in the computer, calculates a relative measurement of the mobility and/or size of the organisms and determines the number thereof by comparison with a previously recorded image and with at least one quotient formation using the time interval between the two images.

3. A device according to claim 1, characterised in that the evaluation system (10') firstly marks in the image objects detected with zero relative mobility and secondly eliminates these in the computer.

4. A device according to claim 1, characterised in that the imaging unit (10) has a single CCD-shutter camera which is moved in cycles over the respective group of test objects (100) under the same conditions.

5. A device according to claim 1, characterised in that the imaging unit (10) has two CCD-shutter cameras, the optical axes of which are arranged at least at an acute angle to one another and determine the movement and/or size of the test organisms in three dimensions.

6. A device according to claim 1, characterised in that the imaging unit (10) is stationary and in that it simultaneously detects a plurality of groups of test organisms (100) under different conditions and sends them for evaluation.

7. A device according to claim 1, characterised in that the imaging unit (10) is stationary and in that a plurality of groups of test organisms (100) under different conditions are moved past the imaging unit (10) at a constant speed and in that the imaging unit (10) sequentially detects these groups and sends them for evaluation.

8. A device according to claim 1, characterised in that groups of test organisms (100) under different conditions are moved in a circle below the imaging unit.

9. A method of operating a device according to at least one of claims 1 to 8, characterised in that the change in the speed of the test organism, established per time interval, is evaluated as a change in mobility.

10. A method of operating a device according to at least one of claims 1 to 8, characterised in that water fleas such as *Daphnia magna* or *Daphnia pulex* are used as a test organism.

11. A method of operating a device according to at least one of claims 1 to 8, characterised in that simulated seawater or freshwater is used as an aqueous medium.

12. A method of operating a device according to at least one of claims 1 to 8, characterised in that the change in mobility and/or size of the test organism is observed in a microtitration plate.

13. A method of operating a device according to at least one of claims 1 to 8, characterised in that the toxic substances are added by pipetting controlled via a display screen.

14. Use of the device according to any one of claims 1 to 8 for determining toxic effects on water fauna.

15. Use of the device according to any one of claims 1 to 8 for determining the effect of toxic substances such as insecticides and pesticides on ecology.

16. Use of the device according to any one of claims 1 to 8 for determining side effects and contra-indications of pharmaceuticals and foodstuffs.

**Revendications**

1. Dispositif pour la détermination de la toxicité de substances solubles dans l'eau ou miscibles à l'eau, dans lequel sont faites au moins une mesure de référence et au moins une autre mesure après l'addition des substances toxiques et l'activité de mouvement d'organismes d'essai est observée par voie optoélectronique perpendiculairement à la surface du milieu aqueux, caractérisé par le fait qu'un organe de prise de vues (10) effectue à des intervalles de temps pouvant être fixés à l'avance une reconnaissance d'objet et un enregistrement séquentiel de la position de l'objet (100), et qu'après cet organe de prise de vues (10) est placé un dispositif d'exploitation (10') comportant un ordinateur et des appareils de présentation qui effectuent une exploitation statistique de la mobilité et/ou de la grandeur des organismes, en relation avec la toxicité existante.

2. Dispositif selon la revendication 1, caractérisé par le fait que l'organe de prise de vues (10) prend une vue en demi-teintes d'au moins un groupe d'organismes d'essai (100) se trouvant dans les mêmes conditions, et que le dispositif d'exploitation (10') qui suit détermine à partir de là des valeurs binaires noir-blanc et effectue une exploitation séquentielle de vue individuelle avec une indication de la position momentanée en coordonnées des organismes d'essai, qui calcule dans le calculateur, par une comparaison avec une vue prise auparavant et au moins une formation de quotient avec l'intervalle de temps entre les deux vues, une mesure relative de la mobilité et/ou de la grandeur des organismes et détermine le nombre de ceux-ci.

3. Dispositif selon la revendication 1, caractérisé par le fait que le dispositif d'exploitation (10') d'une part marque dans l'image et d'autre part élimine dans le calculateur les objets saisis dont la mobilité relative est nulle.

4. Dispositif selon la revendication 1, caractérisé par le fait que l'organe de prise de vues (10) présente une caméra unique à CCD à obturateur qui passe en cadence, dans les mêmes conditions, devant le groupe d'objets d'essai (100).

5. Dispositif selon la revendication 1, caractérisé par le fait que l'organe de prise de vues (10) présente deux caméras à CCD à obturateur dont les axes optiques font au moins un angle aigu et qui saisissent le mouvement et/ou la grandeur des organismes d'essai en trois dimensions.

6. Dispositif selon la revendication 1, caractérisé par le fait que l'organe de prise de vues (10) est fixe et saisit et envoie à l'exploitation simultanément plusieurs groupes d'organismes d'essai (100) se trouvant dans des conditions différentes.

7. Dispositif selon la revendication 1, caractérisé par le fait que l'organe de prise de vues (10) est fixe, que plusieurs groupes d'organismes d'essai (100) se trouvant dans des conditions différentes passent devant celui-ci à une vitesse continue, et que celui-ci saisit et envoie à l'exploitation ces groupes séquentiellement.

8. Dispositif selon la revendication 1, caractérisé par le fait que des groupes d'organismes d'essai (100) se trouvant dans des conditions différentes passent circulairement sous l'organe de prise de vues.

9. Procédé d'exploitation d'un dispositif selon au moins une des revendications 1 à 8, caractérisé par le fait que comme variation de la mobilité est évaluée la variation observée par intervalle de temps de la vitesse de l'organisme d'essai.

10. Procédé d'exploitation d'un dispositif selon au moins une des revendications 1 à 8, caractérisé par le fait que comme organisme d'essai sont employées des daphnies telles que Daphnia magna ou Daphnia pulex.

11. Procédé d'exploitation d'un dispositif selon au moins une des revendications 1 à 8, caractérisé par le fait que comme milieu aqueux est employée de l'eau de mer ou douce artificielle.

12. Procédé d'exploitation d'un dispositif selon au moins une des revendications 1 à 8, caractérisé par le fait que la variation de la mobilité et/ou de la grandeur de l'organisme d'essai est observée dans une plaque de microtitrage.

13. Procédé d'exploitation d'un dispositif selon au moins une des revendications 1 à 8, caractérisé par le fait que l'addition des substances toxiques est effectuée par pipetage contrôlé sur un écran.

14. Utilisation du dispositif selon l'une des revendications 1 à 8 pour la détermination d'effets toxiques sur la faune aquatique.

15. Utilisation du dispositif selon l'une des revendications 1 à 8 pour la détermination de l'influence de substances toxiques telles qu'insecticides et pesticides sur l'écologie.

16. Utilisation du dispositif selon l'une des revendications 1 à 8 pour la détermination d'effets secondaires et de contre-indications de produits pharmaceutiques et de produits alimentaires.

FIG. 1

FIG. 2

FIG. 3

FIG. 4

FIG. 5

EP 0 752 101 B1

St

↓

B-S

↓

Ro

↓

O-P1

↓

O-E1

↓

O-CL

↓

$O-\bar{v}$

↓

R(xi, yi)

↓

O-P2

↓

O-E2

↓

O-CD

↓

RES

FIG. 6

FIG. 7

FIG. 8

FIG. 9

FIG. 10

FIG. 11

## FIG. 12

A

| I | II | III | IV | V |

t [h]

max. 24h

## FIG. 13

A

IV.

1h

t [h]

## FIG.14

A

Pl 01  Pl 02  Pl 03  Pl 04  Pl 05  Pl 06    IV.

10'      10'    t  [min]

## FIG.15

A                                    IV.

a1    a2    a3    a4    a5

10'                                t  [min]

## FIG. 16

IV.

A

a4    a5    a6    a7

t[ms]

20ms

## FIG. 17

IV.

A

S1    S2    S3    S4    S5    S6

t[min]